# EUROPEAN PATENT APPLICATION

(11) **EP 4 393 425 A1**
(43) Date of publication of application: **03.07.2024**
(21) Application number: 23219684.0
(22) Date of filing: 22.12.2023
(51) Int. Cl.: A61B 18/14

(54) **DEFORMED SPINE ELECTRODE BASKET AND METHODS OF THE SAME**

(30) Priority: 27.12.2022 US 202263477315 P; 02.11.2023 US 202318500285
(71) Applicant: Biosense Webster (Israel) Ltd., 2066717 Yokneam (IL)
(72) Inventor: BEECKLER, Christopher Thomas, Irvine, 92618 (US); KHAN, Ishan, Irvine, 92618 (US); OKARSKI, Kevin Mark, Irvine, 92618 (US); GOVARI, Assaf, 2066717 Yokneam (IL)
(74) Representative: Carpmaels & Ransford LLP

(57) **Abstract**

The disclosed technology includes a medical probe including an expandable basket assembly. The basket assembly includes a spine, and the spine includes a deformed region, a spine protrusion, that can be used to deposit an electrode. The electrode can be formed of an electrode assembly that can include an insulative layer and an electrode layer. The electrode assembly is deposed on the spine protrusion so as to avoid the need of separate electrode components that must be positioned along the length of the spine.

## Description

### CROSS-REFERENCE TO RELATED APPLICATIONS

This application claims benefit of, and priority under 35 U.S.C. § 119(e) to, U.S. Provisional Application No. 63/477,315, filed on December 27, 2022, titled "DEFORMED SPINE ELECTRODE BASKET AND METHODS OF THE SAME," which is incorporated by reference in its entirety.

### FIELD

The present invention relates generally to medical devices, and in particular catheters with electrodes, and further relates to, but not exclusively, catheters suitable for use to induce irreversible electroporation (IRE) of cardiac tissues.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Many current ablation approaches in the art tend to utilize radiofrequency (RF) electrical energy to heat tissue. RF ablation can have certain drawbacks, such as heightened risk of thermal cell injury which can lead to tissue charring, burning, steam pop, phrenic nerve palsy, pulmonary vein stenosis, and esophageal fistula. Cryoablation is an alternative approach to RF ablation that generally reduces thermal risks associated with RF ablation. Maneuvering cryoablation devices and selectively applying cryoablation, however, is generally more challenging compared to RF ablation; therefore cryoablation is not viable in certain anatomical geometries which may be reached by electrical ablation devices.

Some ablation approaches use irreversible electroporation (IRE) to ablate cardiac tissue using nonthermal ablation methods. IRE delivers short pulses of high voltage to tissues and generates an unrecoverable permeabilization of cell membranes. Delivery of IRE energy to tissues using multi-electrode catheters was previously proposed in the patent literature. Examples of systems and devices configured for IRE ablation are disclosed in U.S. Patent Pub. No. 2021/0169550A1, 2021/0169567A1, 2021/0169568A1, 2021/0161592A1, 2021/0196372A1, 2021/0177503A1, and 2021/0186604A1, each of which are incorporated herein by reference and attached in the Appendix of priority application no. 63/477,315.

Regions of cardiac tissue can be mapped by a catheter to identify the abnormal electrical signals. The same or different catheter can be used to perform ablation. Some example catheters include a number of spines with electrodes positioned thereon. The electrodes are generally attached to the spines and secured in place by soldering, welding, or using an adhesive. Due to the small size of the spines and the electrodes, however, soldering, or welding the separate electrode components to the spines can be a difficult task, increasing the manufacturing time and cost and the chances that the electrode fails due to an improper bond or misalignment. What is needed, therefore, are systems and methods for producing a spine of a basket assembly without the need for soldering, welding, or using separate electrode attachments to create the electrode assemblies on the spines of the basket assembly.

### SUMMARY

There is provided, in accordance with an embodiment of the present invention, an expandable basket assembly for a multi-electrode catheter. The basket assembly can include at least one spine extending along a longitudinal axis. The at least one spine can be configured to bow radially outward from the longitudinal axis when the expandable basket assembly is transitioned from a collapsed form to an expanded form. The at least one spine can include a spine width and a spine height. The basket assembly can include a spine protrusion having a protrusion width and a protrusion height. At least one of the protrusion width or the protrusion height can be greater than at least one of the spine width and the spine height. The basket assembly can include an electrode assembly being positioned at the spine protrusion. The electrode assembly can include an electrode layer and an insulating layer, the insulating layer being disposed between the electrode layer and the spine protrusion.

The electrode layer can include a film deposited onto the insulating layer. Further, the electrode layer can include a vapor-deposited conductive film. In some examples, electrode layer can include a platinum layer of at least 5 microns nominally in thickness.

The insulating layer can include a film deposited onto the spine protrusion. In some examples, the insulating layer can include a film-cast insulative film. The insulating layer can include, for example, a layer of polyimide, or polyurethane.

The protrusion height can include a height greater than the spine height. The protrusion width can include a width greater than the spine width. The spine protrusion and electrode assembly can form a generally trapezoidal profile. The generally trapezoidal profile of the spine protrusion can include a protrusion length and the electrode assembly can include an electrode assembly length shorter than the protrusion length.

The spine protrusion and electrode assembly can define a curvilinear profile. The spine protrusion and the electrode assembly can form a substantially curvilinear profile having a generally flat surface at a top of each of the electrode assemblies.

The electrode layer can include a portion electrically connected to a medical probe with an electrical connector, and the electrical connector can include a wire. At least a portion of the electrical connector can include an electrically conductive core material comprising a first electrical conductivity, and an electrically conductive cover material comprising a second electrical conductivity less than the first electrical conductivity. The electrically conductive cover material can circumscribe the electrically conductive core material, and an insulative jacket can circumscribe the electrically conductive cover material.

The electrode layer can include a portion electrically connected a medical probe with an electrical connector, and the electrical connector can include a flexible circuit. The flexible circuit can include a printed conductive wire.

The at least one spine can include at least one of carbon fiber, nitinol or cobalt chromium.

The electrode assembly can be configured to deliver electrical pulses for irreversible electroporation, the pulses having a peak voltage of at least 900 volts (V).

The expandable basket assembly can include at least three spines extending radially from an intersection, and each of the at least three spines can include a respective electrode assembly.

The basket assembly can include a tubular shaft including a proximal end and a distal end. The expandable basket assembly can be positioned distal to the distal end of the tubular shaft.

There is also provided, in accordance with an embodiment of the present invention, an expandable basket assembly for a multi-electrode catheter. The method can include forming a first spine for the expandable basket assembly such that a portion of the first spine includes a first spine protrusion. The method can include depositing a first electrode assembly including a first electrode layer covering a portion of the first spine protrusion. The method can include electrically connecting the first electrode layer to an electrical connector of a medical probe.

In some examples, the method can include depositing a first insulating layer of the first electrode assembly onto the first spine protrusion such that the first insulating layer is deposited between the first spine protrusion and the first electrode layer.

In some examples, the method can include depositing the first insulating layer includes vapor-depositing or film-casting the first insulating layer onto the first spine protrusion, and depositing the first electrode layer includes vapor-depositing the first electrode layer onto the first insulating layer.

In some examples, the method can include depositing the first insulating layer includes vapor-depositing or film-casting the first insulating layer onto the first spine protrusion.

The first insulating layer can include a film layer. The first insulating layer can include layer of polyimide.

The electrical connector can include a flexible circuit. The flexible circuit can include a printed conductive wire.

The first spine can include a spine width and a spine height. The first spine protrusion can include a protrusion width and a protrusion height, and at least one of the protrusion width or the protrusion height can be greater than the comparable at least one of the spine width and the spine height.

The first spine protrusion and the first electrode assembly can form a generally trapezoidal profile. The generally trapezoidal profile of the first spine protrusion can include a protrusion length and the first electrode assembly can include an electrode assembly length shorter than the protrusion length.

The first spine protrusion and the first electrode assembly can form a curvilinear profile. The first spine protrusion and the first electrode assembly can form a substantially curvilinear profile having a flat surface at a top of each of the electrode assemblies.

In some examples, the method can include forming a second spine protrusion on the first spine, depositing a second electrode assembly including a second electrode layer proximate the second spine protrusion, electrically connecting the second electrode layer to the electrical connector of the medical probe. The second electrode assembly can include a second insulating layer deposited onto the second spine. The second electrode layer can be deposited on the second insulating layer.

### BRIEF DESCRIPTION OF THE DRAWINGS

FIG. 1 is a schematic pictorial illustration of a medical system comprising a medical probe whose distal end comprises a basket assembly with electrodes, in accordance with an embodiment of the present invention;
FIG. 2A is a schematic pictorial illustration showing a perspective view of a medical probe in an expanded form, in accordance with an embodiment of the present invention;
FIG. 2B is a schematic pictorial illustration showing a side view of a medical probe in a collapsed form, in accordance with the disclosed technology;
FIGs. 3A and 3B are schematic pictorial illustrations showing exploded views of a tubular shaft and spines of the basket assembly to illustrate how the spines can be assembled together with the tubular shaft, in accordance with an embodiment of the present invention;
FIG. 4A is a schematic pictorial illustration showing a side view of a spine, spine protrusion, and electrode assembly, in accordance with an embodiment of the present invention;
FIG. 4B is a schematic pictorial illustration showing a side view of a spine, spine protrusion, and electrode assembly, in accordance with an embodiment of the present invention;
FIG. 4C is a schematic pictorial illustration showing a side view of a spine, spine protrusion, and electrode assembly, in accordance with an embodiment of the present invention;
FIG. 4D is a schematic pictorial illustration showing a top plan view of a spine, spine protrusion, and electrode assembly, in accordance with an embodiment of the present invention; and
FIG. 5 is a flowchart illustrating a method of constructing an expandable basket assembly, in accordance with an embodiment of the present invention.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%. In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "operator" can include a doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The terms "ablate" or "ablation" also include known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode having a high current density and high electric flux density is positioned at a treatment site, and a second electrode having comparatively lower current density and lower electric flux density is positioned remotely from the treatment site.

As discussed herein, the terms "biphasic pulse" and "monophasic pulse" refer to respective electrical signals. "Biphasic pulse" refers to an electrical signal having a positive-voltage phase pulse (referred to herein as "positive phase") and a negative-voltage phase pulse (referred to herein as "negative phase"). "Monophasic pulse" refers to an electrical signal having only a positive or only a negative phase. Preferably, a system providing the biphasic pulse is configured to prevent application of a direct current voltage (DC) to a patient. For instance, the average voltage of the biphasic pulse can be zero volts with respect to ground or other common reference voltage. Additionally, or alternatively, the system can include a capacitor or other protective component. Where voltage amplitude of the biphasic and/or monophasic pulse is described herein, it is understood that the expressed voltage amplitude is an absolute value of the approximate peak amplitude of each of the positive-voltage phase and/or the negative-voltage phase. Each phase of the biphasic and monophasic pulse preferably has a square shape having an essentially constant voltage amplitude during a majority of the phase duration. Phases of the biphasic pulse are separated in time by an interphase delay. The interphase delay duration is preferably less than or approximately equal to the duration of a phase of the biphasic pulse. The interphase delay duration is more preferably about 25% of the duration of the phase of the biphasic pulse.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

The term "temperature rating", as used herein, is defined as the maximum continuous temperature that a component can withstand during its lifetime without causing thermal damage, such as melting or thermal degradation (e.g., charring and crumbling) of the component.

The present disclosure is related to systems, method or uses and devices which utilize end effectors having electrodes affixed to spines. Example systems, methods, and devices of the present disclosure may be particularly suited for IRE ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation.

RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore cryoablation is not viable in certain anatomical geometries which may be reached by electrical ablation devices.

IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

Electroporation can be induced by applying a pulsed electric field across biological cells to cause reversable (temporary) or irreversible (permanent) creation of pores in the cell membrane. The cells have a transmembrane electrostatic potential that is increased above a resting potential upon application of the pulsed electric field. While the transmembrane electrostatic potential remains below a threshold potential, the electroporation is reversable, meaning the pores can close when the applied pulse electric field is removed, and the cells can self-repair and survive. If the transmembrane electrostatic potential increases beyond the threshold potential, the electroporation is irreversible, and the cells become permanently permeable. As a result, the cells die due to a loss of homeostasis and typically die by apoptosis. Generally, cells of differing types have differing threshold potential. For instance, heart cells have a threshold potential of approximately 500 V/cm, whereas for bone it is 3000 V/cm. These differences in threshold potential allow IRE to selectively target tissue based on threshold potential.

The solution of this disclosure includes systems and methods for applying electrical signals from catheter electrodes positioned in the vicinity of myocardial tissue, preferably by applying a pulsed electric field effective to induce electroporation in the myocardial tissue. The systems and methods can be effective to ablate targeted tissue by inducing irreversible electroporation. In some examples, the systems and methods can be effective to induce reversible electroporation as part of a diagnostic procedure. Reversible electroporation occurs when the electricity applied with the electrodes is below the electric field threshold of the target tissue allowing cells to repair. Reversible electroporation does not kill the cells but allows a physician to see the effect of reversible electroporation on electrical activation signals in the vicinity of the target location. Example systems and methods for reversible electroporation is disclosed in U.S. Patent Publication 2021/0162210, the entirety of which is incorporated herein by reference and attached in the Appendix of priority application no. 63/477,315.

The pulsed electric field, and its effectiveness to induce reversible and/or irreversible electroporation, can be affected by physical parameters of the system and biphasic pulse parameters of the electrical signal. Physical parameters can include electrode contact area, electrode spacing, electrode geometry, etc. examples presented herein generally include physical parameters adapted to effectively induce reversible and/or irreversible electroporation. Biphasic pulse parameters of the electrical signal can include voltage amplitude, pulse duration, pulse interphase delay, inter-pulse delay, total application time, delivered energy, etc. In some examples, parameters of the electrical signal can be adjusted to induce both reversible and irreversible electroporation given the same physical parameters. Examples of various systems and methods of ablation including IRE are presented in U.S. Patent Publications 2021/0169550A1, 2021/0169567A1, 2021/0169568A1, 2021/0161592A1, 2021/0196372A1, 2021/0177503A1, and 2021/0186604A1, the entireties of each of which are incorporated herein by reference and attached in the Appendix of priority application no. 63/477,315.

To deliver pulsed field ablation (PFA) in an IRE (irreversible electroporation) procedure, electrodes should contact the tissue being ablated with a sufficiently large surface area. As described hereinbelow, the medical probe includes a flexible insertion tube having proximal and distal ends, and a basket assembly at the distal end of the flexible insertion tube. The basket assembly includes at least one spine and at least one electrode assembly on a deformed section of the elongated spine.

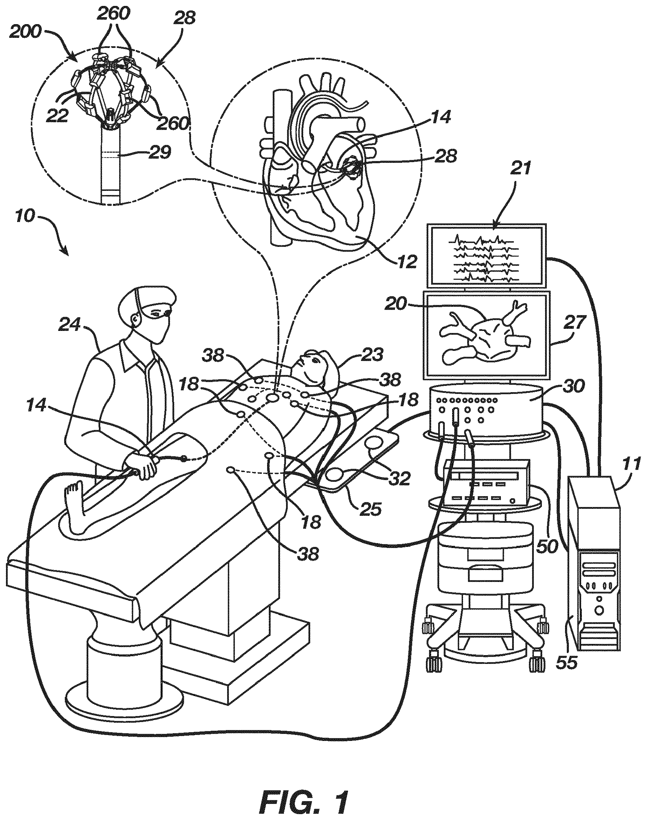

FIG. 1 is a schematic pictorial illustration of a medical system comprising a medical probe whose distal end comprises a basket assembly with electrodes, in accordance with an embodiment of the present invention. The figure shows an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example medical probe 14 (e.g., ablation catheter) that is configured for sensing IEGM is illustrated herein. Physician 24 brings a distal tip 28 of medical probe 14 into contact with the heart wall for sensing a target site in heart 12. For ablation, physician 24 would similarly bring a distal end of an ablation catheter to a target site for ablating.

Medical probe 14 is an exemplary catheter that includes one and preferably multiple electrodes 260 optionally distributed over a plurality of spines 22 at distal tip 28 and configured to sense the IEGM signals. Medical probe 14 may additionally include a position sensor 29 embedded in or near distal tip 28 for tracking position and orientation of distal tip 28. Optionally and preferably, position sensor 29 is a magnetic based position sensor including three magnetic coils for sensing three-dimensional (3D) position and orientation.

Magnetic based position sensor 29 may be operated together with a location pad 25 including a plurality of magnetic coils 32 configured to generate magnetic fields in a predefined working volume. Real time position of distal tip 28 of medical probe 14 may be tracked based on magnetic fields generated with location pad 25 and sensed by magnetic based position sensor 29. Details of the magnetic based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; and 6,892,091.

System 10 includes one or more electrode patches 38 positioned for skin contact on patient 23 to establish location reference for location pad 25 as well as impedance-based tracking of electrodes 260. For impedance-based tracking, electrical current is directed toward electrodes 260 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182.

A recorder 11 displays electrograms 21 captured with body surface ECG electrodes 18 and intracardiac electrograms (IEGM) captured with electrodes 260 of medical probe 14. Recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (5) displaying on display device 27 sites of interest such as places where ablation energy has been applied. One commercial product embodying elements of the system 10 is available as the CARTO^{®} 3 System, available from Biosense Webster, Inc., 31 Technology Drive, Suite 200, Irvine, CA 92618 USA.

It will be noted that the distal tip 28 of medical probe 14 shown in FIG. 1 includes a plurality spines 22 extending outwardly and into a basket shape. The example basket shape shown in FIG. 1 is merely illustrative. The description that follows provides additional details on how the distal tip 28 of the medical probe 14 can be configured.

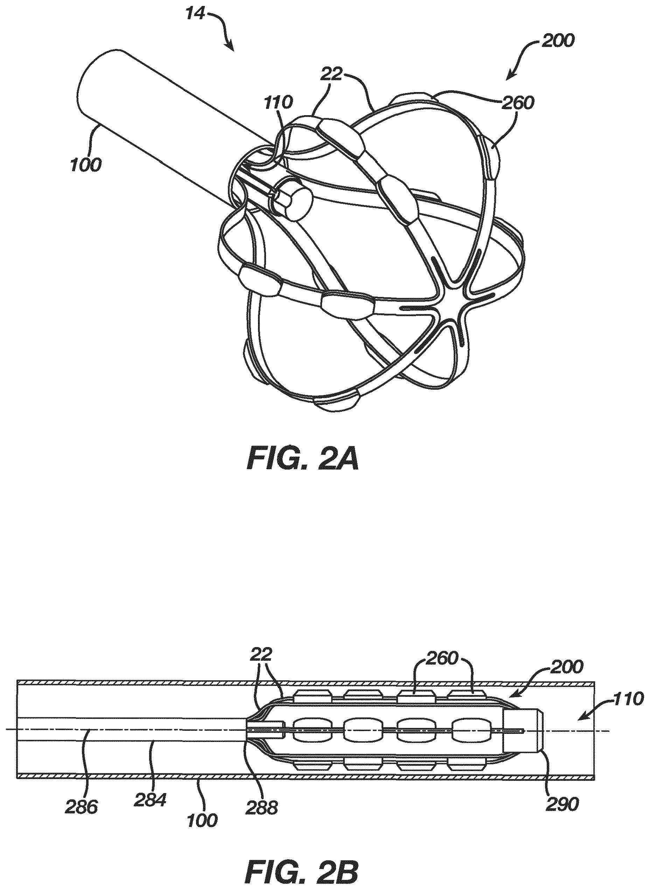

FIG. 2A is a schematic pictorial illustration showing a perspective view of a medical probe 14 with a basket assembly 200 in an expanded form when unconstrained, such as by being advanced out of an insertion tube lumen 110 at a distal end 288 of an insertion tube 100. FIG. 2B shows the basket assembly in a collapsed form within insertion tube 100. In the expanded form (FIG. 2A), the spines 22 bow radially outwardly and in the collapsed form (FIG. 2B) the spines are arranged generally along a longitudinal axis 286 of insertion tube 100.

As shown in FIG. 2A, basket assembly 200 includes a plurality of flexible spines 22 that are formed at the end of a tubular shaft 284 and are connected at both ends. During a medical procedure, medical professional (e.g., physician 24) can deploy basket assembly 200 by extending tubular shaft 284 from insertion tube 100 causing the basket assembly 200 to exit the insertion tube and transition to the expanded form. Spines 22 may have elliptical (e.g., circular) or rectangular (that may appear to be flat) cross-sections, and include a flexible, resilient material (e.g., a shape-memory alloy such as nickel-titanium (also known as Nitinol) or cobalt chromium), or any other suitable material including but not limited to carbon fiber. In some examples, the spines 22 can include an insulative coating such as a polytetrafluoroethylene (PTFE) or Polyether block amide (Pebax^{®}) shrink sleeve, a polyimide film, and/or a coating created by a surface passivation process to further reduce the conductive properties of the spines 22.

In some examples, the spines 22 can include carbon fiber. Carbon fiber can be advantageous in that it is strong, flexible, and provides little hysteresis while being less conductive as compared to Nitinol, for example. An additional advantage of carbon fiber is that epoxy and other adhesives can adhere well to the material, and this can be used to attach electrodes 260 (and/or electrode layers 404 or insulation layers 402) to the spines 22. Additional advantages include high stiffness, high tensile strength, high strength to volume ratio, high chemical resistance, high temperature tolerance, and low thermal expansion. The fibers have a defined direction that can be manipulated to obtain a high degree of flexibility in a particular bending direction.

Referring to the electrodes 260, electrodes 260 can be configured to deliver ablation energy (RF and/or IRE) to tissue in heart 26. Additionally, or alternatively, the electrodes 260 can also be used to determine the location of basket assembly 200 and/or to measure a physiological property such as local surface electrical potentials at respective locations on tissue in heart 26. The electrodes 260 can be biased such that a greater portion of the electrode 260 faces outwardly from the basket assembly 200 such that the electrodes 260 deliver a greater amount of electrical energy outwardly away from the basket assembly 200 (i.e., toward the heart tissue) than inwardly toward the basket assembly 200. This outward flare will be described in greater detail herein with respect to spine protrusions 400.

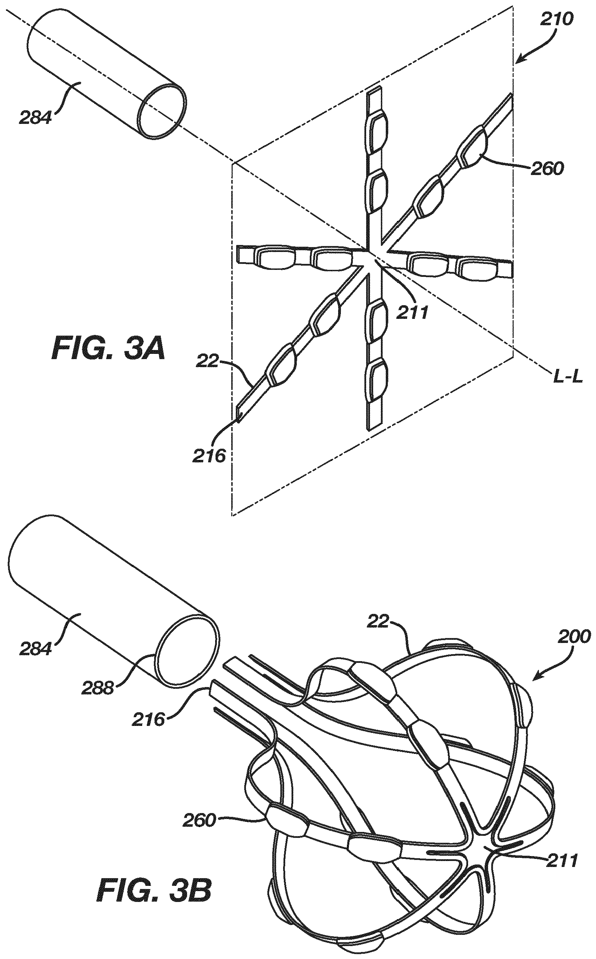

FIGs. 3A and 3B are schematic pictorial illustrations showing exploded views of a tubular shaft 284 and spines 22 of the basket assembly 200 to provide one example of how the spines 22 can be assembled together with the tubular shaft 284, in accordance with an embodiment of the present invention. As shown in FIG. 3A, the spines 22 can be formed from a single sheet of planar material to form a generally star shape. In other words, the spines 22 can be formed from the single sheet of planar material, expect for the spine protrusions 400 described below, such that the spines 22 converge toward a central intersection 211. The intersection 211 can be a solid piece of material (as shown in FIG. 3A) or include one or more apertures (as shown in FIG. 3B). As shown, the basket assembly 200 can have a plurality of spines 22 connected at the intersection 211, including at least three spines, such as the example showing six spines in the figures.

The spines 22 can be folded or otherwise bent such that a proximal end 216 of the spines 22 can be inserted into the distal end 85 of the tubular shaft 284 as shown in FIG. 3B. Although not shown in FIGs. 3A and 3B, it will be appreciated that the electrodes 260 can be attached to or formed on the spines 22 before the spines are inserted into the tubular shaft 284 to form the basket assembly 200. As stated previously, the spines 22 can include a flexible, resilient material (e.g., carbon fiber, nitinol, cobalt chromium, carbon fiber etc.) that can enable the basket assembly 200 to transition from its collapsed form (as shown in FIG. 2B) to its expanded form (as shown in FIG. 2A) when the basket assembly 200 is deployed from insertion tube 100. As will become apparent throughout this disclosure, the spine 22 can be electrically isolated from the electrode 260 to prevent arcing from the electrode 260 to the spine 22.

As will be appreciated by one skilled in the art with the benefit of this disclosure, the basket assembly 200 shown in FIGs. 2A-3B having spines 22 formed from a single sheet of material and converging at a central intersection is offered merely for illustrative purposes, and the disclosed technology can be applicable to other configurations of basket assemblies 200. For example, the disclosed technology can be applicable to basket assemblies 200 formed from a single spine 22 or multiple spines 22 with each spine 22 being attached at both ends. In other examples, the basket assembly 200 can include a central hub connecting the multiple spines 22 together at a distal end 290 of the basket assembly 200. In yet other examples, the basket assembly 200 can include a single spine 22 configured to form a spiral, multiple spines 22 configured to form a spiral, multiple spines 22 configured to form a tripod or multiple tripods, or any other shape of basket assembly 200. As well, the spine assembly 210 can be formed by laser cutting a cylindrical hollow stock material whereby the laser is mounted for rotation about the longitudinal axis (and translation thereto) of the cylindrical stock while cutting through the cylindrical stock. Thus, although FIGs. 2A-3B illustrate a specific configuration of basket assembly 200, the disclosed technology should not be construed as so limited.

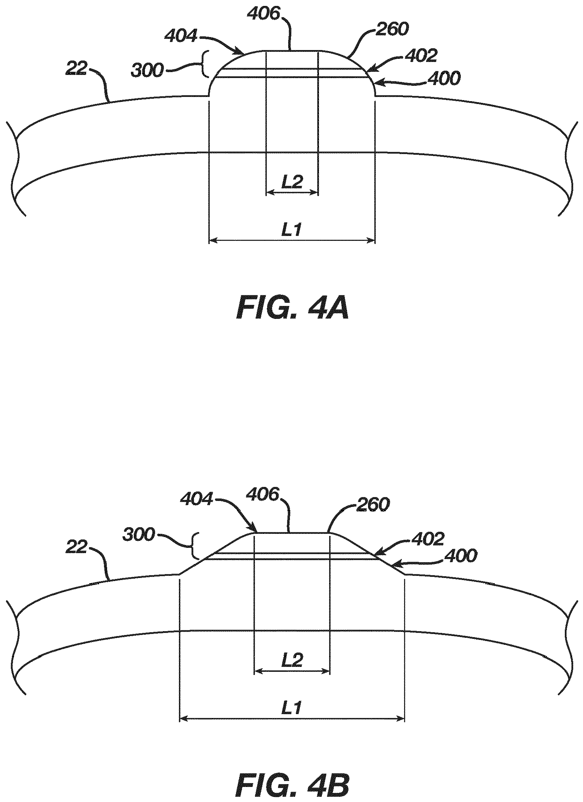

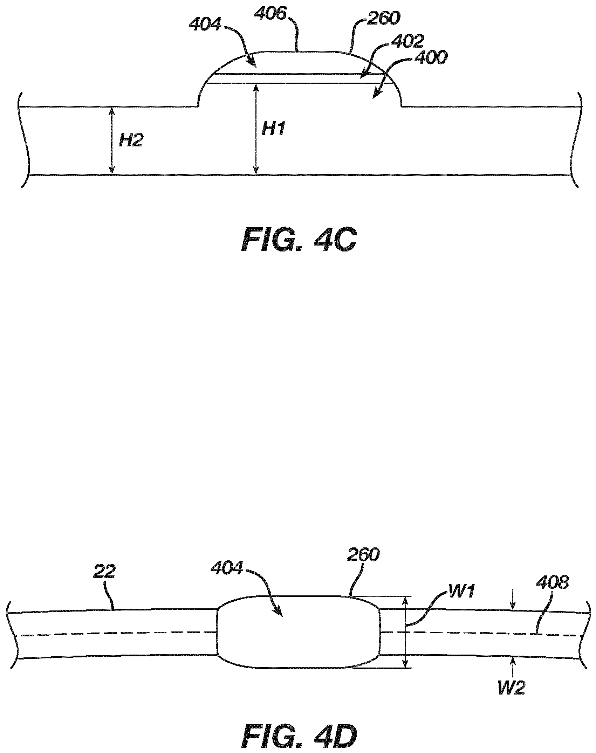

Turning now to FIGs. 4A-4D, various examples of a spine 22, spine protrusion 400, and electrode assembly 300 will be described. As described above, one aim for the electrodes 260 is to bias them outwardly to deliver a greater amount of electrical energy outwardly away from the basket assembly 200 (i.e., toward the heart tissue) than inwardly toward the basket assembly 200. To do so, the electrodes 260 can be formed as an electrode assembly 300 (discussed in greater detail below) placed on a spine protrusion 400. Turning first to the spine protrusion 400, each of the one or more spines 22 of the basket assembly 200 can have one or more spine protrusions 400 extending from the surface of the spine 22. The spine protrusions 400 are deformations in the geometry of the otherwise uniform spine, deformations that provide an area to place an electrode 260 (or an electrode assembly 300). The spine protrusion 400 can have a protrusion width W1 (see FIG. 4D) and a protrusion height H1 (see FIG. 4C). The protrusion width W1 can be greater than the spine width W2 to provide an outward flare such that the electrode 260 has more surface area to contact target tissue. The protrusion height H1 can be greater than the spine height H2 to provide an upward and outward flare toward the target tissue to better contact the tissue.

A benefit of the deformed spine 22, i.e. the spine protrusion 400, is that it provides an area to deposit an electrode 260 directly onto the spine 22 without the need to assemble separate electrode components onto the spines 22. For example, in prior designs, electrode assemblies were separate components that needed to be slid down to their correct positions along the length of the spine 22. Special tooling may be necessary to complete such a manufacturing procedure. With the current design, the electrodes can be manufactured directly onto the spine protrusions 400, which provides significantly improved manufacturing efficiency.

Referring now to the electrode assembly 300, the electrode that is formed on the spine 22 can be formed and/or deposited directly onto the aforementioned spine protrusions 400. The electrode assembly 300 formed onto the respective spine protrusion 400 can include an electrode layer 404 and an insulation layer 402. The insulation layer 402, for example, can be placed between the electrode layer 404 and the spine protrusion 400. Referring first to the insulating layer 402, the layer can be a thin film deposited onto the spine protrusion 400. This film can be deposited, for example, via film-casting or vapor deposition onto the spine protrusion 400. To use an example, an insulative material can be film-casted in a thin film layer on the spine protrusion 400 so as to create the insulation layer 402 upon which the electrode layer 404 is deposited. The insulative material can be, for example, polyetherimide (PEI), polyimide, polyetheretherketon (PEEK), polytetraflouroethylene (PTFE), fluorinated ethylene propylene (FEP), polyurethane, etc. In some examples, the insulation layer 402 can be an epoxy that adheres the electrode layer 404 to the spine protrusion 400.

Referring now to the electrode layer 404, the layer can be a film layer deposited onto the basket assembly 200. In some examples, the film layer can be deposited onto the insulation layer 402. It will be appreciated, however, in some examples the electrode assembly 300 may only include the electrode layer 404, for example in scenarios where the spines 22 are made out of non-conductive materials such as carbon fiber. In that use case, the electrode layer 404 can be deposited directly onto the spine protrusions 400.

The electrode layer 404 can include a vapor-deposited conductive film. In some examples, the electrode layer 404 can include gold, platinum, palladium, and/or their respective alloys. These materials also have high thermal conductivity which allows the minimal heat generated on the tissue (i.e., by the ablation energy delivered to the tissue) to be conducted through the electrodes to the back side of the electrodes (i.e., the portions of the electrodes adjacent the spines 22), and then to the blood pool in heart 26. Considering platinum as an example material, the platinum layer for the electrode layer 404 can be at least 5 microns nominally in thickness.

Referring now to the shape of the spine protrusions 400 and electrode assemblies 300 shown in FIGs. 4A and 4B, the profile of the spine protrusions 400 and electrode assemblies 300 can be smooth so as to provide an atraumatic profile. Accordingly, the ends of the spine protrusions 400 and electrode assemblies 300 along the longitudinal axis of the spines 22 can have a smooth taper towards a top/tip 406 of the electrode assemblies 300. Considering FIG. 4A for example, the spine protrusion 400 and electrode assembly 300 can define a curvilinear profile. The profile of the spine protrusion 400 can have a base protrusion length L1, and the profile of the spine protrusion 400 can taper inwardly toward the insulation layer 402, and the insulation layer 402 can taper inwardly to the electrode layer 404. The top/tip 406 of the electrode layer can have a generally flat surface having a length L2 shorter than the base protrusion length L1. Referring now to FIG. 4B, the spine protrusion 400 and electrode assembly 300 can form a generally trapezoidal profile. Similar to the curvilinear profile, the generally trapezoidal profile of the spine protrusion 400 can have a protrusion length L1, and the electrode assembly 300 can have an electrode assembly length L2 shorter than the protrusion length L1.

Referring now to FIG. 4D, which is a top plan view of an example spine 22 with a deformed area (protrusion) to provide a base for an electrode assembly 300. The electrode layer 404 can include an electrical connection to the medical probe 14 via an electrical connector 408. The electrical connector can include a wire connecting the electrode layer 404 to the medical probe 14. In other examples, the electrical connector 408 can include a flexible circuit. In one example, the flexible circuit can be an electrical connection created via photolithography, depositing the flexible circuit directly onto the spine 22, or the electrical connection can be separated from the spine 22 by an insulation layer. The flexible circuit can also be a conductive film sputtered directly onto the spine 22, or the film can be separated from the spine 22 by an insulation layer. In yet other examples, the flexible circuit can be a printed conductive wire printed directly onto the spine 22, or the printed wire can be separated from the spine 22 by an insulation layer. The flexible circuit and/or wire can also be covered by an insulation layer such that the current passing through the electrical connector 408 is not dissipated into surrounding tissue before reaching the electrode 260.

In any example, the electrical connector 408 can have an electrically conductive core material comprising a first electrical conductivity, and an electrically conductive cover material comprising a second electrical conductivity less than the first electrical conductivity. The electrically conductive cover material can circumscribe the electrically conductive core material, and an insulative jacket can circumscribe the electrically conductive cover material. The electrical connector 408 in any example can be configured to withstand a voltage difference of adjacent wires of the basket assembly 200 sufficient to deliver IRE pulses. Preferably, the electrical connector 408 can withstand at least 900V, and more preferably at least 1800V between adjacent wires. Further, in all examples, the electrical connector 408 itself can deliver electrical pulses having a peak voltage of at least 900V.

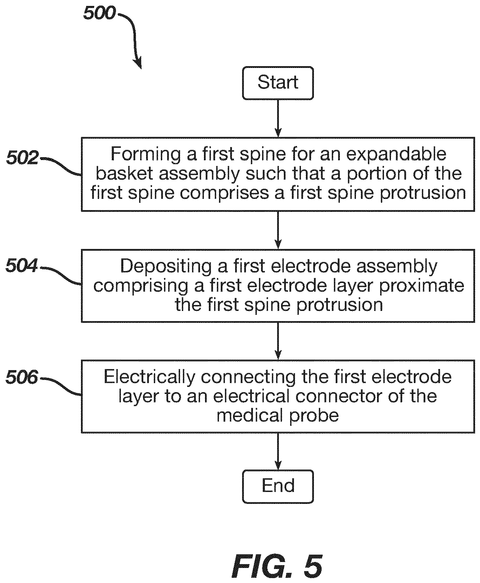

FIG. 5 is a flowchart illustrating a method 500 of constructing an expandable basket assembly 200, in accordance with an embodiment of the present invention. The method 500 can include forming 502 a first spine 22 for the expandable basket assembly 200 such that a portion of the first spine 22 comprises a first spine protrusion 400. The method 500 can include depositing 504 a first electrode assembly 300 comprising a first electrode layer 404 covering a portion of the first spine protrusion 400. The method 500 can include electrically connecting 506 the first electrode layer 404 to an electrical connector 408 of a medical probe 14.

The method 500 can end after the connecting 506 step. In other examples, additional steps to construct the expandable basket 200 can be performed. For example, method 500 can include depositing a first insulating layer 402 of the first electrode assembly 300 onto the first spine protrusion 400 such that the first insulating layer 402 is deposited between the first spine protrusion 400 and the first insulating layer 402. Depositing the first insulating layer can include film-casting (or vapor depositing) the first insulating layer 402 onto the first spine protrusion 400, and depositing the first electrode layer 404 can include vapor-depositing the first electrode layer 404 onto the first insulating layer 402. Depositing the first insulating layer 402 can include film-casting (or vapor-depositing) the first insulating layer 402 onto the first spine protrusion 400.

Further, the method 500 can include forming a second spine protrusion on the first spine 22. The method 500 can include depositing a second electrode assembly comprising a second electrode layer proximate the second spine protrusion. The method 500 can include electrically connecting the second electrode layer to the electrical connector of the medical probe.

Examples of the present disclosure can be implemented by any of the following numbered clauses:
Clause 1: An expandable basket assembly comprising: at least one spine extending along a longitudinal axis and configured to bow radially outward from the longitudinal axis when the expandable basket assembly is transitioned from a collapsed form to an expanded form, the at least one spine comprising a spine width and a spine height; a spine protrusion comprising a protrusion width and a protrusion height, at least one of the protrusion width or the protrusion height is greater than at least one of the spine width and the spine height; and an electrode assembly being positioned at the spine protrusion and comprising: an electrode layer; and an insulating layer disposed between the electrode layer and the spine protrusion.
Clause 2: The assembly according to Clause 1, the electrode layer includes a film deposited onto the insulating layer.
Clause 3: The assembly according to Clause 1 or Clause 2, the electrode layer includes a vapor-deposited conductive film.
Clause 4: The assembly according to Clause 1 or Clause 2, the electrode layer comprising a platinum layer of at least 5 microns nominally in thickness.
Clause 5: The assembly according to any of Clauses 1-4, the insulating layer includes a film deposited onto the spine protrusion.
Clause 6: The assembly according to any of Clauses 1-5, the insulating layer includes a vapor-deposited insulative film.
Clause 7: The assembly according to any of Clause 1-6, the insulating layer includes a film-cast insulative film.
Clause 8: The assembly according to any of Clauses 1-7, the insulating layer includes a layer of polyimide.
Clause 9: The assembly according to any of Clauses 1-8, the protrusion height comprises a height greater than the spine height.
Clause 10: The assembly according to any of Clauses 1-9, the protrusion width includes a width greater than the spine width.
Clause 11: The assembly according to any of Clauses 1-10, the spine protrusion and electrode assembly form a generally trapezoidal profile.
Clause 12: The assembly according to Clause 11, the generally trapezoidal profile of the spine protrusion comprises a protrusion length and the electrode assembly comprises an electrode assembly length shorter than the protrusion length.
Clause 13: The assembly according to any of Clauses 1-10, the spine protrusion and electrode assembly defining a curvilinear profile.
Clause 14: The assembly according to any of Clauses 1-10, spine protrusion and the electrode assembly form a substantially curvilinear profile having a generally flat surface at a top of each of the electrode assemblies.
Clause 15: The assembly according to any of Clauses 1-14, the electrode layer includes a portion electrically connected to a medical probe with an electrical connector, and the electrical connector includes a wire.
Clause 16: The assembly according to Clause 15, at least a portion of the electrical connector comprises an electrically conductive core material comprising a first electrical conductivity, an electrically conductive cover material comprising a second electrical conductivity less than the first electrical conductivity, the electrically conductive cover material circumscribing the electrically conductive core material, and an insulative jacket circumscribing the electrically conductive cover material.
Clause 17: The assembly according to any of Clauses 1-14, the electrode layer includes a portion electrically connected a medical probe with an electrical connector, and the electrical connector includes a flexible circuit.
Clause 18: The assembly according to Clause 17, the flexible circuit comprises a printed conductive wire.
Clause 19: The assembly according to any of Clauses 1-18, the at least one spine comprises at least one of carbon fiber, nitinol or cobalt chromium.
Clause 20: The assembly according to any of Clauses 1-19, the electrode assembly is configured to deliver electrical pulses for irreversible electroporation, the pulses having a peak voltage of at least 900 volts (V).
Clause 21: The assembly according to any of Clauses 1-20, the expandable basket assembly comprises at least three spines extending radially from an intersection, and each of the at least three spines comprises a respective electrode assembly.
Clause 22: The assembly according to any of Clauses 1-20 further comprising a tubular shaft including a proximal end and a distal end, the expandable basket assembly positioned distal to the distal end of the tubular shaft.
Clause 23: A method of constructing an expandable basket assembly, the method comprising: forming a first spine for the expandable basket assembly such that a portion of the first spine comprises a first spine protrusion; depositing a first electrode assembly comprising a first electrode layer covering a portion of the first spine protrusion; and electrically connecting the first electrode layer to an electrical connector of a medical probe.
Clause 24: The method according to Clause 23 further comprising depositing a first insulating layer of the first electrode assembly onto the first spine protrusion such that the first insulating layer is deposited between the first spine protrusion and the first electrode layer.
Clause 25: The method according to Clause 24, depositing the first insulating layer comprises vapor-depositing the first insulating layer onto the first spine protrusion, and depositing the first electrode layer comprises vapor-depositing the first electrode layer onto the first insulating layer.
Clause 26: The method according to Clause 24, depositing the first insulating layer comprises vapor-depositing the first insulating layer onto the first spine protrusion.
Clause 27: The method according to Clause 24, depositing the first insulating layer comprises film-casting the first insulating layer onto the first spine protrusion.
Clause 28: The method according to any of Clauses 24-27, the first insulating layer includes a film layer.
Clause 29: The method according to any of Clauses 24-28, the first insulating layer includes a layer of polyimide.
Clause 30: The method according to any of Clauses 23-29, the electrical connector includes a flexible circuit.
Clause 31: The method according to Clause 30, the flexible circuit comprises a printed conductive wire.
Clause 32: The method according to any of Clauses 23-31, the first spine comprises a spine width and a spine height, the first spine protrusion comprises a protrusion width and a protrusion height, and at least one of the protrusion width or the protrusion height is greater than the comparable at least one of the spine width and the spine height.
Clause 33: The method according to any of Clauses 23-32, the first spine protrusion and the first electrode assembly form a generally trapezoidal profile.
Clause 34: The method according to Clause 33, the generally trapezoidal profile of the first spine protrusion comprises a protrusion length and the first electrode assembly comprises an electrode assembly length shorter than the protrusion length.
Clause 35: The method according to any of Clauses 23-34, the first spine protrusion and the first electrode assembly form a curvilinear profile.
Clause 36: The method according to any of Clauses 23-34, the first spine protrusion and the first electrode assembly form a substantially curvilinear profile having a flat surface at a top of each of the electrode assemblies.
Clause 37: The method according to any of Clauses 23-36 further comprising: forming a second spine protrusion on the first spine; depositing a second electrode assembly comprising a second electrode layer proximate the second spine protrusion; and electrically connecting the second electrode layer to the electrical connector of the medical probe.
Clause 38: The method according to Clause 37, the second electrode assembly comprises a second insulating layer deposited onto the second spine, the second electrode layer deposited on the second insulating layer.

The embodiments described above are cited by way of example, and the present invention is not limited by what has been particularly shown and described hereinabove. Rather, the scope of the invention includes both combinations and sub combinations of the various features described hereinabove, as well as variations and modifications thereof which would occur to persons skilled in the art upon reading the foregoing description and which are not disclosed in the prior art.

## Claims

1. An expandable basket assembly comprising:
at least one spine extending along a longitudinal axis and configured to bow radially outward from the longitudinal axis when the expandable basket assembly is transitioned from a collapsed form to an expanded form, the at least one spine comprising a spine width and a spine height;
a spine protrusion comprising a protrusion width and a protrusion height, at least one of the protrusion width or the protrusion height is greater than at least one of the spine width and the spine height; and
an electrode assembly being positioned at the spine protrusion and comprising:
an electrode layer; and
an insulating layer disposed between the electrode layer and the spine protrusion.

2. The assembly according to claim 1, the electrode layer includes a film deposited onto the insulating layer.

3. The assembly according to claim 1 or claim 2, wherein the electrode layer includes a vapor-deposited conductive film or a platinum layer of at least 5 microns nominally in thickness.

4. The assembly according to any preceding claim 1, the insulating layer includes a film deposited onto the spine protrusion.

5. The assembly according to any preceding claim, the insulating layer includes a vapor-deposited insulative film.

6. The assembly according to any preceding claim, the insulating layer includes a film-cast insulative film.

7. The assembly according to any preceding claim, the insulating layer includes a layer of polyimide.

8. The assembly according to any preceding claim, the protrusion height comprises a height greater than the spine height.

9. The assembly according to any preceding claim, the protrusion width includes a width greater than the spine width.

10. The assembly according to any preceding claim, the spine protrusion and electrode assembly form a generally trapezoidal profile., optionally wherein the generally trapezoidal profile of the spine protrusion comprises a protrusion length and the electrode assembly comprises an electrode assembly length shorter than the protrusion length.

11. The assembly according to any of claims 1 to 9, the spine protrusion and electrode assembly i) defining a curvilinear profile, or ii) forming a substantially curvilinear profile having a generally flat surface at a top of each of the electrode assemblies.

12. The assembly according to any preceding claim, the electrode layer includes a portion electrically connected to a medical probe with an electrical connector, and the electrical connector includes a wire, optionally at least a portion of the electrical connector comprises an electrically conductive core material comprising a first electrical conductivity, an electrically conductive cover material comprising a second electrical conductivity less than the first electrical conductivity, the electrically conductive cover material circumscribing the electrically conductive core material, and an insulative jacket circumscribing the electrically conductive cover material.

13. The assembly according to any of claims 1 to 11, the electrode layer includes a portion electrically connected a medical probe with an electrical connector, and the electrical connector includes a flexible circuit, optionally wherein the flexible circuit comprises a printed conductive wire.

14. A method of constructing an expandable basket assembly, the method comprising:
forming a first spine for the expandable basket assembly such that a portion of the first spine comprises a first spine protrusion;
depositing a first electrode assembly comprising a first electrode layer covering a portion of the first spine protrusion; and
electrically connecting the first electrode layer to an electrical connector of a medical probe.

15. The method according to claim 14 further comprising depositing a first insulating layer of the first electrode assembly onto the first spine protrusion such that the first insulating layer is deposited between the first spine protrusion and the first electrode layer.
